# EUROPEAN PATENT APPLICATION

(11) **EP 4 799 679 A1**
(43) Date of publication of application: **02.09.2026**
(21) Application number: 25160239.7
(22) Date of filing: 26.02.2025
(51) Int. Cl.: A61N 5/06

(54) **APPARATUS FOR PROVIDING CONTROLLED EMISSION OF LIGHT AND SYSTEM USING THE SAME**

(71) Applicant: 24Lumo Healthcare UG, 55411 Bingen am Rhein (DE)
(72) Inventor: HOHENSEE, Günter, 21077 Hamburg (DE); AKKERMANS, Ton, 5583 XG Waalre (NL); FALCK, Thomas, 52066 Aachen (DE)
(74) Representative: Hoffmann Eitle

(57) **Abstract**

The present invention relates to an apparatus for providing controlled emission of light and a system including the same, and in particular to an apparatus including several light sources and a controller. The apparatus controls the emission of PWM blue light at approximately 40 Hz and white light such as to reduce the perception of flickering by visual masking of the 40 Hz light.

## Description

### Field of the Invention

The present invention relates to an apparatus for providing controlled emission of light and a system using the same, and particularly to an apparatus comprising several light sources emitting light at different wavelengths.

### Background

In recent years, research into the application of light therapies for neurological diseases, particularly Alzheimer's disease (AD), has made significant progress. A promising scheme involves the use of pulsed blue light, e.g. with a wavelength of 473 nm and modulated at a frequency of 40 Hz, to stimulate neuronal activity and reduce amyloid plaques in the brain.

EP 3 383 482 B1 explains that key microscopic pathological hallmarks of AD include the presence of amyloid plaques and extensive neuronal loss. This accumulation of neuronal insults occurs over a length of time and induces macroscopic circuit dysfunctions in the brain, specifically gamma power deficits during memory and attention tasks. Gamma oscillations primarily originate, and are modulated by, fast-spiking-parvalbumin (FS-PV)-interneurons. EP 3 383 482 B1 uses these findings and describes systems for preventing, mitigating, and treating dementia in a subject comprising inducing synchronized gamma oscillations in at least one brain region of the subject by applying light, sound or/and a haptic stimulus.

Cristina Blanco-Duque et al. present in the Journal of Internal Medicine an article entitled "Audiovisual gamma stimulation for the treatment of neurodegeneration", published February 2024, Volume 295; pages 146-170. They discuss the therapeutic potential of gamma stimulation in treating AD and the mechanisms responsible for its positive effects. Recent studies have been presented showing that it is feasible and safe to induce 40 Hz brain activity in AD patients through a range of 40 Hz multisensory and noninvasive electrical or magnetic stimulation methods. These studies suggest that 40 Hz stimulation can yield beneficial effects on brain function, disease pathology, and cognitive function in individuals with AD. Studies are discussed involving 40 Hz light, auditory, and vibrotactile stimulation, as well as noninvasive techniques, such as transcranial alternating current stimulation and transcranial magnetic stimulation. These studies suggest that neuronal and non-neuronal mechanisms may be involved, touching upon the relevance of interneurons, neuropeptides, and specific synaptic mechanisms in translating gamma stimulation into widespread neuronal activity within the brain.

Accordingly, methods and systems are under investigation for preventing, mitigating, and/or treating dementia in a subject, which include controlling a stimulus-emitting device to emit a stimulus and exposing the subject to the stimulus and/or administering the stimulus to the subject in order to induce in vivo synchronized gamma oscillations in at least one brain region of the subject. The stimulus may have a frequency of about 35 Hz to about 45 Hz, such as a frequency of about 40 Hz.

However, there are several disadvantages specifically with the type of visual stimulations in the conventional art:
1. Limited Patient Tolerance: Existing light therapy systems often do not offer the ability to adjust parameters of stimulating light pulses, such as the intensity, duty cycle or frequency, by taking the individual tolerance levels of the patient into account. This can lead to discomfort, unwanted side effects, or even discontinuation of the therapy.
2. Risk of Macular Degeneration: A significant issue of existing light therapy systems is the potential risk of inducing macular degeneration. This risk is particularly pronounced when light intensity and spectrum are not properly managed. Factors such as luminance, the distance between the light source and the patient, and the spectrum used can play a critical role in patient safety. Inadequate control of these parameters can cause oxidative damage to the photoreceptors in the retina, leading to long-term vision problems.
3. Unpleasant Perception Due to Flickering: Therapies often apply a visually observable frequency, such as 40 Hz, of pulsed light. This frequency can be perceived by the patient as unpleasant flickering, which can cause discomfort and potentially reduce therapy adherence. Such flickering can be especially bothersome during longer therapy sessions, negatively impacting on the patient's well-being.
4. Limited Flexibility in Light Representation: Systems are often restricted to a fixed color spectrum and rigid light modulations, limiting the effectiveness of the therapy, as not all patients respond equally well to the same frequencies and wavelengths.

### Summary

Therefore, an aim is to provide a novel apparatus for providing controlled emission of light and a system using the same able to address one or more of the above disadvantages.

According to an embodiment, an apparatus for providing controlled emission of light comprises two light sources and a controller. The controller is coupled to the first light source and the second light source. The first light source is configured to emit first light. The first light emitted by the first light source has a wavelength in the range between 460 to 485nm, preferably between 470nm and 475nm. For example, the first light source emits light with a center wavelength of 473nm. The second light source is configured to emit second light. The second light emitted by the second light source is visible light having a broad spectrum of wavelengths spanning at least 250nm in the wavelength range between 400nm and 700nm. The broad wavelength spectrum spectrally overlaps with the wavelength of the first light source. The controller is configured to control the first light source so that the first light is pulse width modulated with a frequency between 30Hz and 50Hz, preferably between 35Hz and 45Hz, such as 40Hz, and configured to change a duty cycle of the pulse width modulated first light. The controller is further configured to control the second light source to emit the second light at the same time as the first light, i.e., the emissions overlap in time so that the first light and the second light illuminate an area in a patient's eye.

Accordingly, unpleasant flickering of the first light can be suppressed by the second light which is perceived by the patient at the same time. In other words, reduction of perceived flickering is enabled by utilizing second light which overlaps in time with the first light, e.g. because it uses a much higher frequency than the pulsed first light. This reduction in flickering improves the comfort and acceptance of therapy, making the overall light appear more stable and less intrusive.

According to an advantageous example, the broad spectrum of wavelengths of the second light is chosen so that the second light is perceived as white light by the patient, or it is chosen so that the second light is perceived as colored light. Accordingly, the perception of flickering is reduced by visual masking of the approximately 40 Hz pulses of the first light in the blue wavelength region with second light constituting colored, e.g. bluish or greenish, or white light. Visual masking increases the acceptance of patients because the prominence of flickering is reduced.

According to an advantageous example, the second light source comprises a plurality of light emitting diodes (LEDs) having different wavelength spectra emitting a continuous spectrum of wavelengths, and/or the first light source comprises a plurality of light emitting diodes having the same wavelength spectrum. Accordingly, second light may be generated by different LEDs used to obtain a broad spectrum perceivable as white light, e.g. with RGB LEDs and/or warm and/or cold white LEDs so as to flexibly mask the flickering of the first light generated by the same LEDs all emitting in the blue region.

According to an advantageous example, the second light source is adjustable to emit the second light with a color temperature ranging from 2700K to 6500K. Accordingly, the apparatus may also provide a circadian cycle, a natural oscillation that repeats roughly every 24 hours, providing warm white light in the morning and evening, and cold white light temperatures around noon time.

According to an advantageous example, the apparatus further comprises a third light source including red, blue and green light emitting diodes (RGB LEDs) and wherein the second light source comprises warm white and cold white light emitting diodes so as to be adjustable to emit the light with a color temperature ranging from 2700K to 6500K. Accordingly, there is the flexibility to provide a white light background at different light temperatures and/or to display colored moving objects or images using RGB LEDs which distracts the patient from the flickering first light.

According to an advantageous example, the controller is configured to control the second light source so that the second light is pulse width modulated, preferably with a frequency between 5kHz and 50kHz, most preferably with a frequency of 25kHz. Accordingly, the intensity can be controlled smoothly by changing the duty cycle. In particular, the frequency of the pulse width modulated second light is chosen to be magnitudes higher than the frequency of the first light, which ensures that the first and second lights overlap in time.

According to an advantageous example, the apparatus further comprises one or more sensors configured to measure one or more parameters, including luminance, intensity, power, wavelength spectrum and/or source-target distance. Accordingly, negative side effects of the light therapy can be mitigated, such as the risk of macular degeneration. That is, relevant parameters, such as luminance, spectrum, and distance from the light sources, and especially of the first light source, can be monitored and adjusted, e.g. by applying an algorithm guaranteeing that light intensity thresholds are not exceeded, thus ensuring safe application.

According to an advantageous example, the controller is configured to control the first light source in a first operation phase by increasing the power of the first light and maintaining the duty cycle at a constant percentage value above 75%. Accordingly, an acclimatization period with smooth introduction of the 40 Hz light therapy is provided. For example, to make it more pleasant and acceptable for the patient, the 40 Hz first light starts with a longer pulse width of e.g. 80% on and 20% off and adapts slowly to a 40 Hz light being 50% on and 50% off.

According to an advantageous example, the controller is configured to control the first light source in a second operation phase by increasing the power and decreasing the duty cycle so as to maintain approximately the same intensity of the first light, wherein the duty cycle is preferably decreased from a percentage value above 75% to 50%. Accordingly, an acclimatization period with smooth introduction of the 40 Hz light therapy is provided. For example, to make it more pleasant and acceptable for the patient, the 40 Hz first light starts with a longer pulse width of e.g. 80% on and 20% off and adapts slowly to a 40 Hz light being 50% on and 50% off. Depending on the age and acceptance of the patient, this transition period can be adapted in between e.g. 3 and 6 minutes.

According to an advantageous example, the controller is configured to control the first light source in a third operation phase by maintaining the duty cycle at a constant percentage value in a range between 40% and 60%. Accordingly, the light level is also adapted to the same light level for continuous treatment after acclimatization.

According to an advantageous example, the controller is configured to control the first light source in the first operation phase during a first time frame, then in the second operation phase during a second time frame, and then in the third operation phase during a third time frame. Accordingly, an acclimatization period with smooth introduction of the 40 Hz light therapy is provided.

According to an advantageous example, the apparatus comprises a user interface configured to enable the patient or the user to adjust the intensity, power and/or the duty cycle of at least one of the first light and the second light. Accordingly, flexible control of the treatment parameters is possible, which leads to higher acceptance of the therapy. An acclimatization period with smooth introduction of the 40 Hz light therapy is adjustable and provided before a treatment period.

According to an advantageous example, the controller is configured to decrease the intensity of second light if the intensity of the first light is increased, and/or to increase the intensity of second light if the intensity of the first light is decreased so that the overall intensity of the first and second lights remains constant. Accordingly, precise adjustment of light intensity, and pulse on/off cycle adjusted to the individual tolerance thresholds of the patient is possible maintaining overall light exposure below a certain limit. For example, a patient or healthcare professional may adjust using a user interface the intensity of one of the lights and the other light emission is adapted automatically by an intelligent algorithm controlling the light energy based on patient characteristics and light measurements.

According to an embodiment, a system is provided comprising one or more of the apparatuses described above, wherein the first, second, and/or third light source are arranged in a panel. Accordingly, the light emission can be distributed over a large area of a panel which can be easily and effectively used in a bedside setting.

According to an advantageous example, the system comprises an electroencephalography (EEG) device arranged to provide an output to the controller. The controller changes the intensity of the first light source based on this output. Accordingly, the effectiveness of the therapy can be detected, and feedback can be provided to the apparatus. For example, the intensity is increased, and the treatment time is decreased or vice versa based on the EEG device output to provide feedback to the controller. In particular, the intensity of the first light and/or the length of exposure time of the first light can be adjusted.

According to an advantageous example, the panel of the system has a surface area of more than 0.8 square meters and is mountable on a ceiling, wall or rack, wherein one of the second or third light sources comprises red, green and blue light emitting diodes arranged on the panel so that moving images are displayable. Accordingly, an effective system for a hospital or other bedside setting can be provided which can effect first light exposure over a long period of time which can be effectively masked by other light exposures distracting the patient and making therapy more acceptable.

### Brief description of the drawings

Fig. 1 illustrates an apparatus according to an embodiment.
Fig. 2 illustrates a system according to another embodiment.
Figs. 3A, 3B and 3C show different phases of an exemplary operation of the apparatus.
Fig. 4A illustrates PWM signals with different duty cycles.
Fig. 4B illustrates the overlap in time of two light sources.
Fig. 4C illustrates the spectral overlap of two light sources.
Fig. 5 illustrates a lighting panel of a system using the apparatus.

### Detailed description of the preferred embodiments

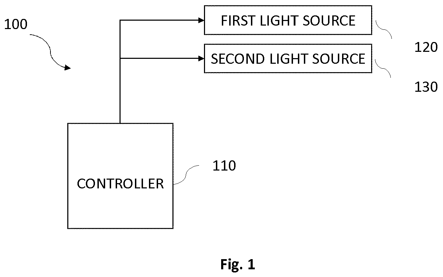

Fig. 1 illustrates an apparatus according to an embodiment. The apparatus 100 emits light to a patient, wherein the emission is controlled by a controller 110. As shown in Fig. 1, controller 110 is coupled to the first light source 120 and the second light source 130.

The first light source 120 emits first light. The first light has a wavelength in the spectral range between 460 to 485nm, preferably a center wavelength between 470nm and 475nm. The first light source may comprise one or more blue LEDs; thus, the wavelength in the spectral range between 460 to 485nm can be considered the center wavelength of an emission peak of a narrow emission spectrum with a full width at half maximum (FWHM) between 10 and 80 nm and preferably 15 and 40 nm. For example, the first light source 120 comprises a plurality of light emitting diodes having the same wavelength spectrum, e.g. a plurality of blue LEDs. In particular, the first light source 120 emits light with a center wavelength peaking at approximately 473nm.

The second light source 130 emits second light. The second light 130 is visible light having a broad spectrum of wavelengths spanning at least 250nm in the wavelength range between 400nm and 700nm. For example, the broad spectrum of wavelengths of the second light is chosen so that the second light is perceived as white light by the patient.

Alternatively, the second light is chosen so that the second light is perceived as colored light, preferably light having an emission peak in the blue and/or green region so as to largely overlap with the light emitted by the first light source 120. In particular, the wavelength range and intensity of the second light is controlled so that the patient cannot consciously easily distinguish between the first and second lights, i.e. that some kind of visual masking is realized.

The second light source 130 is preferably adjustable to emit the second light with a color temperature ranging from 2700K to 6500K.

For example, the second light source 130 comprises a plurality of light emitting diodes having different wavelength spectra emitting a continuous spectrum of wavelengths, e.g. one or more blue and green LEDs or blue (B), green (G) and red (R) LEDs.

Additionally, or alternatively, the second light source 130 comprises a plurality of light emitting diodes having different wavelength spectra emitting a continuous white spectrum of wavelengths, e.g. one or more warm white LEDs and one or more cold white LEDs, which may be based on blue LEDs having some kind of fluorescent and/or luminescent light conversion layer on top of the blue LED chip.

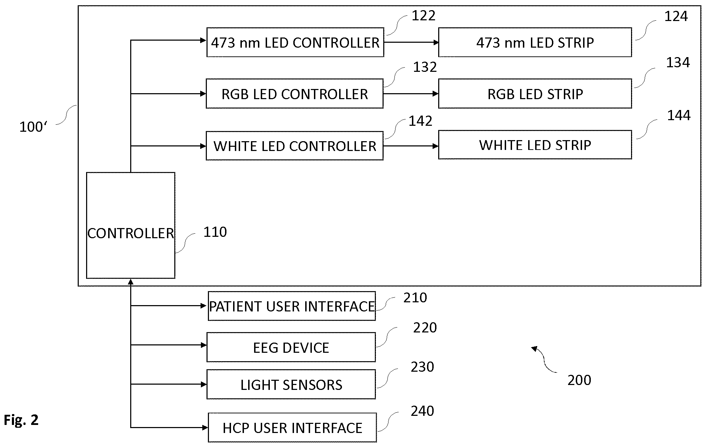

In an example, the apparatus 100 may further comprise a third light source (not shown in Fig. 1), a possible realization of such an apparatus is discussed in Fig. 2. Hence, it is understood that all or only some features of Fig. 2's apparatus could be integrated in Fig. 1's apparatus.

The controller 110 is a light controller coupled to the first light source 120 and the second light source 130 and controls the two light sources. The controller 110 is configured to control the first light source 120 so that the first light is pulse width modulated. Two key parameters control the pulse width modulation (PWM) signal used to drive the light source, the frequency of the switching and the relative duration of the on-time or on-cycle which is referred to duty cycle here. That is, a duty cycle or power cycle is the fraction of one period (the time it takes for a signal to complete an on-and-off cycle) in which a signal or system is active, which is commonly expressed as a percentage or a ratio.

In more detail, the controller 110 is configured to control the first light source 120 so that the first light is pulse width modulated with a frequency between 30Hz and 50Hz.

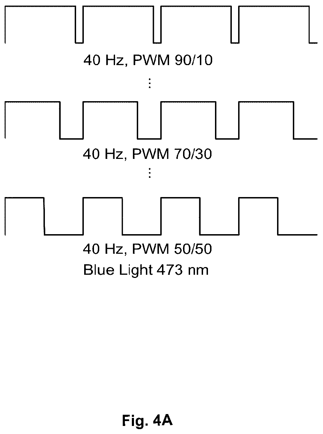

Preferably the frequency is controlled between 35Hz and 45Hz, wherein 40 Hz is currently considered to be the optimal frequency. The controller 110 is further configured to change a duty cycle of the pulse width modulated first light. That is, the controller 110 can switch the PWM signal on and off increasing or decreasing the on cycle according to the desired light intensity. Different duty cycles will later be explained with respect to Fig. 4A.

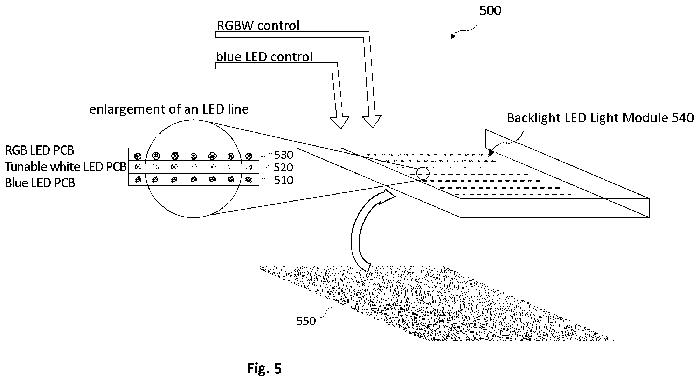

The controller 110 is further configured to control the second light source 130 to emit the second light. The controller 110 controls the second light source so that the second light is emitted at the same time as the first light. In other words, both light emissions overlap in time and are emitted so that the first light and the second light illuminate an area in a patient's eye. That is, the two light sources 120, 130 are arranged in such a way that their emissions go approximately in the same direction. For example, the light sources 120, 130 may be based on LED technology and arranged next to each other in a light module of a lighting panel, an example of which is discussed later with respect to Fig. 5. The light emitted by the light sources of the apparatus enters the patient's eye through the pupil and is incident on the retina; a part of which or all of it may be regarded as an area that is illuminated so as to induce brain activity in the patient.

The controller 110 may be configured to control the second light source so that the second light is pulse width modulated. In particular, the PWM frequency is chosen to be much higher than the frequency of the first light so as to ensure a large temporal overlap between the first and second lights. For example, a frequency between 5kHz and 50kHz is set in the controller, e.g. by a user or as a default by the manufacturer. As a practical example, a frequency of 25kHz may be used.

The controller 110 may be realized by any device or devices capable of processing data by performing logical and/or mathematical operations. For instance, a controller may include a central processing unit, a processor, e.g. microprocessor, or a computer or any other processing logic that may interpret and execute instructions and may form the main part of a controller, such as controller 110. The controller may further include a memory, such as a ROM and/or a RAM, or another type of dynamic storage device that may store information and software instructions for execution by the controller's processing logic.

In particular, where the term controller is used, no restriction is made regarding how distributed this element may be and regarding how gathered these elements may be. That is, the constituent elements may be distributed in different software and hardware components or other elements for bringing about the intended function. A plurality of distinct elements may also be gathered for providing the intended functionalities. For example, the apparatus 100' in Fig. 2 schematically illustrates next to the light controller 110 three individual controllers 122, 132 and 142 for three light sources, respectively. In this context, it should be understood that each of the individual controllers can also be part of the light controller 110 or of the respective light source. In other words, circuitry or processing logic fulfilling the function of such an individual controller can be integrated in the light controller 110, or respective light source, or both.

Fig. 2 illustrates a system 200 including the apparatus 100', which may be the same as apparatus 100 or a modified version thereof. In addition to the first and second light sources 124, 144, the apparatus 100' comprises a third light source 134 including red, blue and green LEDs, for example. The second light source 144 may comprise white light emitting diodes, preferably warm white and cold white LEDs. The second light source 144 may be configured to be adjustable so as to emit light with a color temperature ranging from 2700K to 6500K.

The exemplary system 200 also includes a patient user interface (PUI) 210, electroencephalography (EEG) sensor or device 220, light sensors 230 and/or a health care professional (HCP) user interface 240. These units may be coupled to the controller 110 of the apparatus 100' to input information which can be used for the operation of the apparatus. In another embodiment (not shown), one or more of these units may be part or incorporated into the apparatus 100 or 100'.

As mentioned above, apparatus 100' may include optional individual controllers, such as a 473 nm LED controller 122, an RGB LED controller 132, and a white light LED controller 142. On the one side, these controllers are coupled to the light controller 110 providing control signals to the individual controllers. On the other side, each of these individual controllers is coupled to a respective one of the three light sources, such as the 473 nm LED strip 124, the RGB LED strip 134, and the white LED strip 144. Alternatively, the individual controllers 122, 132, 142 may be integrated in the controller 110 or the light sources 124, 134, 144. The above mentioned PWM control can be performed in any of the described controllers so as to achieve the herein described functions, e.g. different duty cycles and frequencies.

The light sources 124, 134, 144 are not limited to strips, but may also be realized in different arrangements, e.g. the LEDs of the light sources may be arranged differently on one or more printed circuit boards (PCB) or a unit of a light module or luminaire. For example, the LEDs of the light sources may be mixed, e.g. a unit of a light module may form include a plurality of macro pixels each including white light, RGB and 473nm LEDs.

The patient user interface (PUI) 210 may be part of the apparatus 100, 100' or the system 200 and may be configured to enable the patient to adjust the intensity, power and/or the duty cycle of at least one of the first light and the second light. The PUI 210 may comprise a knob to tune RGB color, a knob to set RGB brightness, a knob to tune white color temperature, a knob to set white light brightness, an "I feel uncomfortable"-button and/or a Start-/Stop-Button.

The health care professional (HCP) user interface 240 may be part of the apparatus 100, 100' or the system 200 and may be configured to enable the patient to adjust the intensity, power and/or the duty cycle of at least one of the first light and the second light. The HCP user interface 240 may comprise a knob to tune intensity of 473nm LEDs, e.g. arranged on a strip, and knobs to set the age of a patient, a duration of therapy, and/or a sensitivity of a patient.

Instead of the knobs, the user interfaces above may be any type of input means, such as a button, a control dial, jog dial, a joystick, or a touch screen or portion thereof.

The electroencephalography (EEG) device 220 comprises one or more sensors for detecting brain waves, e.g. electrical signals from the medial entorhinal cortex or hippocampus areas of the person's brain. For example, EEG signals are sensed using conventional EEG detectors/sensors, such as metal electrodes, placed on or near the person's scalp. After the EEG device 220 reads the brain waves, the controller may receive these waves and adjust the duty cycle of the first and/or second light source. That is, the controller is adapted to receive feedback from the EEG device and may thus adjust the intensity of the first light source based on the feedback.

The one or more light sensors 230 measure one or more parameters, including luminance, intensity, power, wavelength spectrum and/or source-target distance. Accordingly, negative side effects of the light therapy can be mitigated, such as the risk of macular degeneration. That is, relevant parameters, such as luminance, spectrum, and distance from the light sources, especially of/from the first light source, can be monitored and adjusted, e.g. by applying an algorithm guaranteeing that light intensity thresholds are not exceeded, ensuring safe application. Hence, it is possible to provide feedback to the apparatus.

As outlined above, the apparatus comprises a first light source 120, 124 that can emit next to the therapeutic 40 Hz light of 473 nm wavelength also white light and/or colored light from a second light source 130, 144 and/or third light source. Calibrated 473 nm LEDs ensure therapeutic efficacy through precise wavelength control.

The light controller 110 may control the light sources based on input from one or more of the sources discussed above:
- Patient input from the PUI 210 allows the patient to customize the system; in particular, the patient can indicate when he/she cannot tolerate the 40 Hz light so that the light controller can reduce the power of the 473 nm light and possibly extend the length of the treatment. Thus, therapy parameters may be adjusted to the individual tolerance of the patient.
- Healthcare professional input from the HCP user interface 240 allows the HCP to control the system. Thus, therapy parameters may be adjusted to the individual tolerance of the patient. This allows also to protect the patient by adhering to safe thresholds for glare and luminance, for example.
- Light measurements from one or more light sensors 230 can provide light intensity to inform the light controller 110 whether the glare is within an acceptable range.
- EEG information of the patient measured and processed by the EEG device 220 can inform the light controller whether the pulsed light, e.g. 40 Hz light, is perceived by the patient, i.e. whether a change of an EEG signal is detected when the 40 Hz light is turned on.

Thus, the apparatus and the system address the insufficient adaptability of existing light therapy devices to individual patient needs and the associated risks, such as the potential induction of macular degeneration. Additionally, the uncomfortable flickering that occurs when using the 40 Hz frequency is effectively reduced by implementing another light source emitting light at the same time, e.g. by a carrier frequency, i.e. a frequency which is much higher than the frequency of the pulsed first light, for example 25 kHz. Hence, the apparatuses and systems discussed offer personalized, flexible, and safe light therapies that are tailored to the specific clinical and technical requirements of each patient, while also maximizing comfort during therapy.

Advantages over existing technologies result from integrating a second or even third light source having a high carrier frequency, such as 25 kHz, which is controlled via Pulse Width Modulation (PWM). The additional light source(s) and the frequencies used not only support the generation of a full color spectrum using RGB LEDs, but also allow for the representation of color temperatures ranging from 2700K to 6500K in the white spectrum. These features are designed to make the therapy more comfortable for patients and to maximize the tolerance. The implementation of the carrier frequency enables a gentler and less intrusive light therapy while maintaining or even improving the effectiveness of the treatment. The one or more integrated user interfaces allow the healthcare professional and/or the patient to control the light therapy themselves. Depending on the individual patient's tolerance and compliance, the patient and/or healthcare professional can adjust or even stop the therapy. An algorithm running in the controller may calculate the pulse length and energy of the 40 Hz light therapy and set the additional white light in such a way that the patient feels comfortable. The apparatus or the system may, depending on the tolerance level and age, change from a very comfortable light setting to the most efficient therapy level during a therapy time.

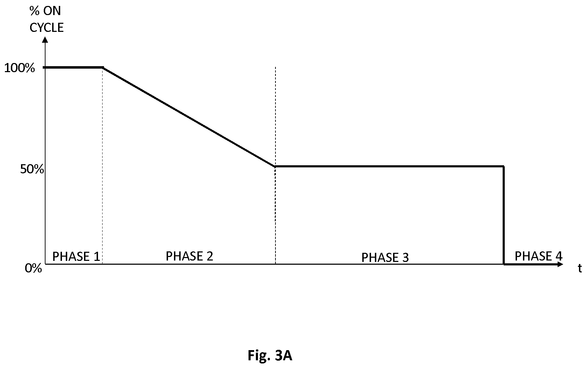

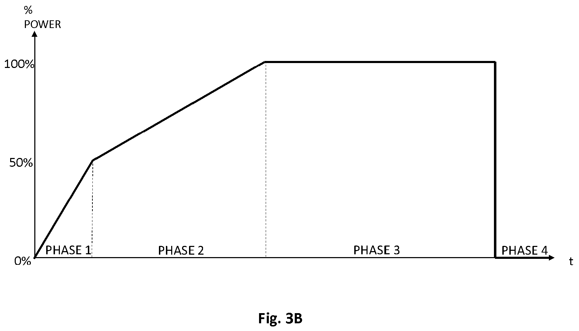

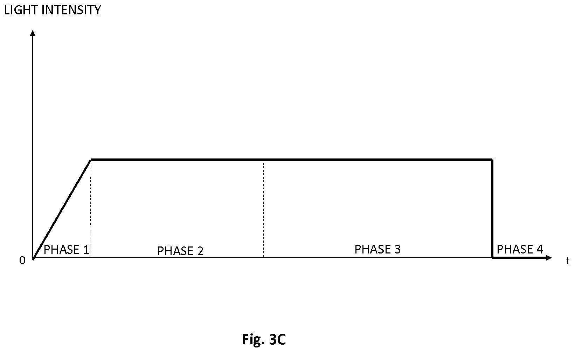

In the following, an exemplary operation of the apparatus 100, 100' is discussed with respect to Figs. 3A, 3B and 3C. Figs. 3A, 3B and 3C show four phases of an exemplary operation and particularly the different settings of duty cycle, power and light intensity in these different phases.

These figures show that the controller 110 divides the light therapy into four phases.

In phase 1, the controller is configured to control the first light source in a first operation phase so as to increase the power of the first light and maintain the duty cycle at a constant percentage value above 75%. Specifically, the figures illustrate in phase 1 ramping up the light intensity of the 473 nm light without flickering. The 473 nm light is regulated up from zero to the target intensity level with a duty cycle of 100%. In addition, the colored light and/or the white light of the second and/or third light sources may be regulated from zero up to the target intensity level.

In phase 2, e.g. an acclimatization period, the controller controls the first light source in a second operation phase so as to increase the power and decrease the duty cycle while maintaining approximately the same intensity of the first light. For example, the duty cycle is preferably decreased from a percentage value above 75% to 50%. Specifically, the figures illustrate that the duty cycle of the 473 nm / 40 Hz light is smoothly reduced from 100% to 50%. The power is adjusted to keep the light intensity on a constant level. An example of a transition of pulses with a high duty cycle of 90% to 70% and then to 50% is illustrated in Fig. 4A, thus illustrating PWM signals with different duty cycles. The skilled person knows that the duty cycle may be reduced in even smaller steps so as to make the change approximately continuous. In addition, the colored light and/or white light continues on the target intensity level.

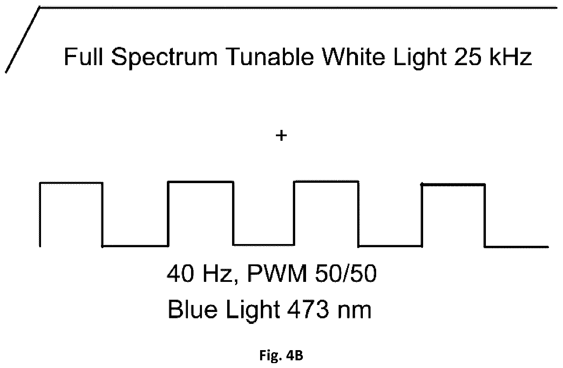

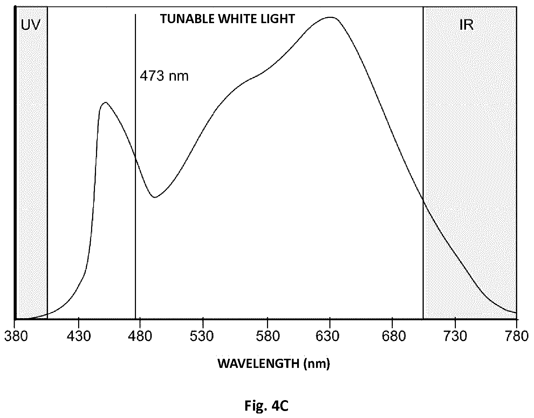

In phase 3, e.g. a therapy period, the controller controls the first light source in a third operation phase by maintaining the duty cycle at a constant percentage value in a range between 40% and 60%. Specifically, the figures illustrate that the 40 Hz / 473 nm light is kept at a duty cycle of approximately 50% with a constant power and thus constant light intensity. Additionally, the colored light and/or white light remains on the target intensity level. An example of applying white light at a constant intensity level combined with first light at a duty cycle of 50% is illustrated in Fig. 4B. Fig. 4B illustrates the overlap in time of two light sources. Fig. 4C illustrates the spectral overlap of two light sources, such as one or more LEDs at 473 nm and white light LEDs, such as warm white and cold white LEDs, which can be tuned to emit white light of a desired light temperature. It is understood that the line for the blue LED in Fig. 4C is merely a schematic illustration and in practice, such as an LED with a peak (center) wavelength around 473nm would usually have a broader, gaussian-like, spectrum.

In phase 4, e.g. an end of therapy, the 473 nm light is switched off. The colored and/or white light can remain on the target intensity level, if desired by the patient, or can simply be switched off.

As explained with respect to Figs 3A, 3B and 3C, the controller controls the first light source in the first operation phase during a first time frame, then in the second operation phase during a second time frame and then in the third operation phase during a third time frame. Accordingly, a start-up period, an acclimatization period and a therapy period may be realized one after the other, wherein the length in time of the periods is depending on the overall dose needed for therapy. In particular, if the 473nm light is not well received by the patient, the intensity of this light can be reduced and the intensity of the second light can be ramped up so that the overall intensity is more or less constant. However, since the intensity of the first light is reduced, the length of the therapy period needs to be increased to reach the same dose.

For example, the controller may decrease the intensity of second light if the intensity of the first light is increased, and/or to increase the intensity of second light if the intensity of the first light is decreased so that the overall intensity of the first and second lights remains constant.

Fig. 5 illustrates a lighting panel 500 of a system, such as system 200, using a lighting apparatus, such as the apparatus 100, 100'. The system's panel 500 can have different shapes, e.g. rectangular or round. For example, the panel 500 has a surface area of more than 0.8 square meters, i.e., comparable or larger than a 55 inch display. Preferably, the surface area is more than 1.5 square meters, e.g. to cover a large part of a wall or ceiling. The panel 500 may be mountable on a ceiling, wall or rack, and may also be curved or divided in two parts, e.g. one on a ceiling and one on a wall. Accordingly, a flexible design can be realized with good usability of the light panel in different spatial orientations, e.g. vertical and horizontal.

The panel 500 comprises an LED light module 540 and a diffuser 550. The diffuser 550 may be a diffuser foil or other diffuser materials, such a light diffusing hard plastic cover, to close off the panel's light module, e.g. the backlight LED light module 540 in Fig. 5. This arrangement using the diffuser 550 can ensure that the light can be largely glare-free.

Thus, the above-described panel 500 allows to emit light over a large area and as glare-free as possible.

The panel may include the first, second and/or third light sources. The panel may comprise a light source including red, green and blue light emitting diodes arranged in the panel so that moving images or objects are displayable. In other words, the RGB LEDs may be arranged to form a display which can be used to display moving images or objects to distract a patient from the light therapy with the pulsed blue light.

The light module 540 of Fig. 5 comprises three light sources, such as RGB LEDs 530, tunable white light LEDs 520 and blue LEDs 510. Each light source may be placed on a printed circuit board (PCB). Each light source may be formed as one or more strips so that the module includes several strips of the RGB LEDs 530, several strips of the tunable white light LEDs 520 and several strips of the blue LEDs 510 which may be arranged alternatingly in or on the panel. A strip may be a line.

As explained above with respect to Fig. 1, the RGB and white light LEDs of the panel may be controlled by the above controller indicated as RGBW control and the blue light LEDs may also be controller by the above controller indicated as blue LED control in Fig. 5.

According to the above, several of the above-mentioned problems in the art are solved.

A large-area light element, such as the panel 500, is realized which utilizes advanced LED technology that is flexibly controlled according to the needs in therapy and tolerance/acceptance of a patient. This element employs specially calibrated LEDs precisely tuned to a wavelength of 473 nm (+/- a minimal tolerance), for example. This calibration is based on the latest research findings and ensures optimal therapeutic efficacy with minimal risk to the patient.

The apparatus' luminance can be carefully limited to ensure that maximum allowable glare thresholds and blue light hazard are not exceeded. This minimizes the risk of eye damage and ensures that the light therapy can be applied safely. In particular, several additional light sources may be applied to increase the patient's acceptance by reducing the perception of flickering.

The light element can be used both vertically and horizontally on a wall, rack and/or ceiling allowing for flexible application in various therapeutic settings. This versatility enables adaptation to the specific needs and preferences of the patient as well as the spatial conditions of the therapy environment.

The system is controlled via a user-friendly interface that can be both optical, e.g. display, and mechanical/electrical, e.g. keys, softkeys, touch screen keys, etc. The interface can be operated directly by the patient and/or HCP and allows for the adjustment of light intensity and parameters to match the patient's maximum tolerance and compliance. This feedback system ensures that therapy parameters are dynamically adjusted to achieve the desired dosage without overwhelming the patient.

It will be apparent to those skilled in the art that various modifications and variations can be made in the entities and methods of this invention as well as in the construction of this invention without departing from the scope of the invention.

The invention has been described in relation to particular embodiments and examples which are intended in all aspects to be illustrative rather than restrictive. Those skilled in the art will appreciate that many different combinations of hardware, software and/or firmware will be suitable for practicing the present invention.

Moreover, other implementations of the invention will be apparent to those skilled in the art from consideration of the specification and practice of the invention disclosed herein. It is intended that the specification and the examples be considered as exemplary only. To this end, it is to be understood that inventive aspects lie in less than all features of a single foregoing disclosed implementation or configuration. Thus, the true scope and spirit of the invention is indicated by the following claims.

## Claims

1. Apparatus for providing controlled emission of light, comprising:
a first light source configured to emit first light, wherein the first light emitted by the first light source has a wavelength in the range between 460 to 485nm, preferably between 470nm and 475nm;
a second light source configured to emit second light, wherein the second light emitted by the second light source is visible light having a broad spectrum of wavelengths spanning at least 250nm in the wavelength range between 400nm and 700nm; and
a controller coupled to the first light source and the second light source,
wherein the controller is configured to control the first light source so that the first light is pulse width modulated with a frequency between 30Hz and 50Hz, preferably between 35Hz and 45Hz, and configured to change a duty cycle of the pulse width modulated first light; and
wherein the controller is further configured to control the second light source to emit the second light at the same time as the first light so that the first light and the second light illuminate an area in a patient's eye.

2. The apparatus according to claim 1, wherein the broad spectrum of wavelengths of the second light is chosen so that the second light is perceived as white light by the patient or is chosen so that the second light is perceived as colored light.

3. The apparatus according to claim 1 or 2, wherein the second light source comprises a plurality of light emitting diodes having different wavelengths spectra emitting a continuous spectrum of wavelengths and/or the first light source comprises a plurality of light emitting diodes having the same wavelength spectrum.

4. The apparatus according to one of claims 1 to 3, wherein the second light source is adjustable to emit the second light with a color temperature ranging from 2700K to 6500K.

5. The apparatus according to one of claims 1 to 4, further comprising a third light source including red, blue and green light emitting diodes and wherein the second light source comprises warm white and cold white light emitting diodes so as to be adjustable to emit the light with a color temperature ranging from 2700K to 6500K.

6. The apparatus according to one of claims 1 to 5, wherein the controller is configured to control the second light source so that the second light is pulse width modulated, preferably with a frequency between 5kHz and 50kHz, most preferably with a frequency of 25kHz.

7. The apparatus according to one of claims 1 to 6, comprising one or more sensors configured to measure one or more parameters, including luminance, intensity, power, wavelength spectrum and/or source-target distance.

8. The apparatus according to one of claims 1 to 7, wherein the controller is configured to control the first light source in a first operation phase by increasing the power of the first light and maintaining the duty cycle at a constant percentage value above 75%.

9. The apparatus according to one of claims 1 to 8, wherein the controller is configured to control the first light source in a second operation phase by increasing the power and decreasing the duty cycle so as to maintain approximately the same intensity of the first light, wherein the duty cycle is preferably decreased from a percentage value above 75% to 50%.

10. The apparatus according to one of claims 1 to 9, wherein the controller is configured to control the first light source in a third operation phase by maintaining the duty cycle at a constant percentage value in a range between 40% and 60%.

11. The apparatus according to claim 10, wherein the controller is configured to control the first light source in the first operation phase during a first time frame, then in the second operation phase during a second time frame and then in the third operation phase during a third time frame.

12. The apparatus according to one of claims 1 to 11, wherein the apparatus comprises a user interface configured to enable the patient or a user to adjust the intensity, power and/or the duty cycle of at least one of the first light and the second light.

13. The apparatus according to one of claims 1 to 12, wherein the controller is configured to decrease the intensity of second light if the intensity of the first light is increased and/or to increase the intensity of second light if the intensity of the first light is decreased so that the overall intensity of the first and second lights remains constant.

14. System comprising one or more of the apparatuses of one of claims 1 to 13, wherein the first, second and/or third light source are arranged in a panel.

15. The system of claim 14, wherein the system comprises an electroencephalography device arranged to provide an output to the controller, and/or
wherein the panel has a surface area of more than 0.8 square meters and is mountable on a ceiling, wall or rack, and wherein one of the second or third light sources comprises red, green and blue light emitting diodes arranged on the panel so that moving images or objects are displayable.
